(19) Europäisches Patentamt
European Patent Office
Office européen des brevets

(11) Publication number: 0 048 001
B1

(12) EUROPEAN PATENT SPECIFICATION

(45) Date of publication of patent specification: 19.12.84

(51) Int. Cl.³: C 07 D 475/08, C 07 D 241/26

(21) Application number: 81107198.4

(22) Date of filing: 11.09.81

# (54) Method for preparing N-(4-(((2,4-diamino-6-pteridinyl)-methyl)methylamino)benzoyl)glutamic acid.

(30) Priority: 12.09.80 DK 3903/80

(43) Date of publication of application:
24.03.82 Bulletin 82/12

(45) Publication of the grant of the patent:
19.12.84 Bulletin 84/51

(84) Designated Contracting States:
AT BE CH DE FR GB IT LI LU NL SE

(56) References cited:
US-A-4 057 548

JOURNAL OF MEDICINAL CHEMISTRY, Vol. 17, No. 11, 1974 M. CHAYKOVSKY et al. "Methotrexate Analogs. 3. Synthesis and Biological Properties of Some Side-Chain Altered Analogs" pages 1212 to 1216

JOURNAL OF ORGANIC CHEMISTRY, Vol. 40, No. 23, 1975 Baltimore H.G. MAUTNER et al. "The Circular Dichroism Spectra of Folic Acid and 10-Thiafolic Acid and the Porblem of Racemization in the Synthesis of Analogs of Folic Acid through the Cyclization of Substituted 2-Amino-3-cyanopyrazines" pages 3447 to 3448

(73) Proprietor: Farmos Group Ltd.
Tengströminkatu 6 P.O. Box 425
SF-20101 Turku 10 (FI)

(72) Inventor: Jensen, Jens Karl
Berggreensgade 26
DK-2100 Copenhagen O (DK)
Inventor: Alster, Karol
Ellegaardspark 44
DK-3520 Farum (DK)
Inventor: Svendsen, Björn
Stenholtsvej 20
DK-3480 Fredensborg (DK)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22 (DE)

EP 0 048 001 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a novel and convenient method for preparing methotrexate, and to an important intermediate useful in the method.

Methotrexate (N-[4-[[(2,4-diamino-6-pteridinyl)methyl]methylamino]benzoyl]glutamic acid) is a well-known antineoplastic agent useful in the treatment of acute leukemia and other cancerous diseases. In the past, methotrexate has been accepted in a rather impure state and with unspecified optical rotation (cf. U.S.P. XIX, 1973—74, p. 315 and B.P. 1973, p. 300).

The synthesis of pure methotrexate is difficult because of the complexity of the molecule and because of the extreme insolubility of the pteridines. However, one known method has proved to be especially good for the preparation of methotrexate as the reactions involved in the method are easy, and as the reaction sequence gives very pure methotrexate, in particular free from its 7-isomer. This method (called "the Taylor method": E.C. Taylor, Chemistry and Biology of Pteridines, Proceedings of the 5th International Symposium, Konstanz, 1975, Walter de Gruyter, Berlin, 1975, pp. 543—573) is illustrated in the following reaction sequence:

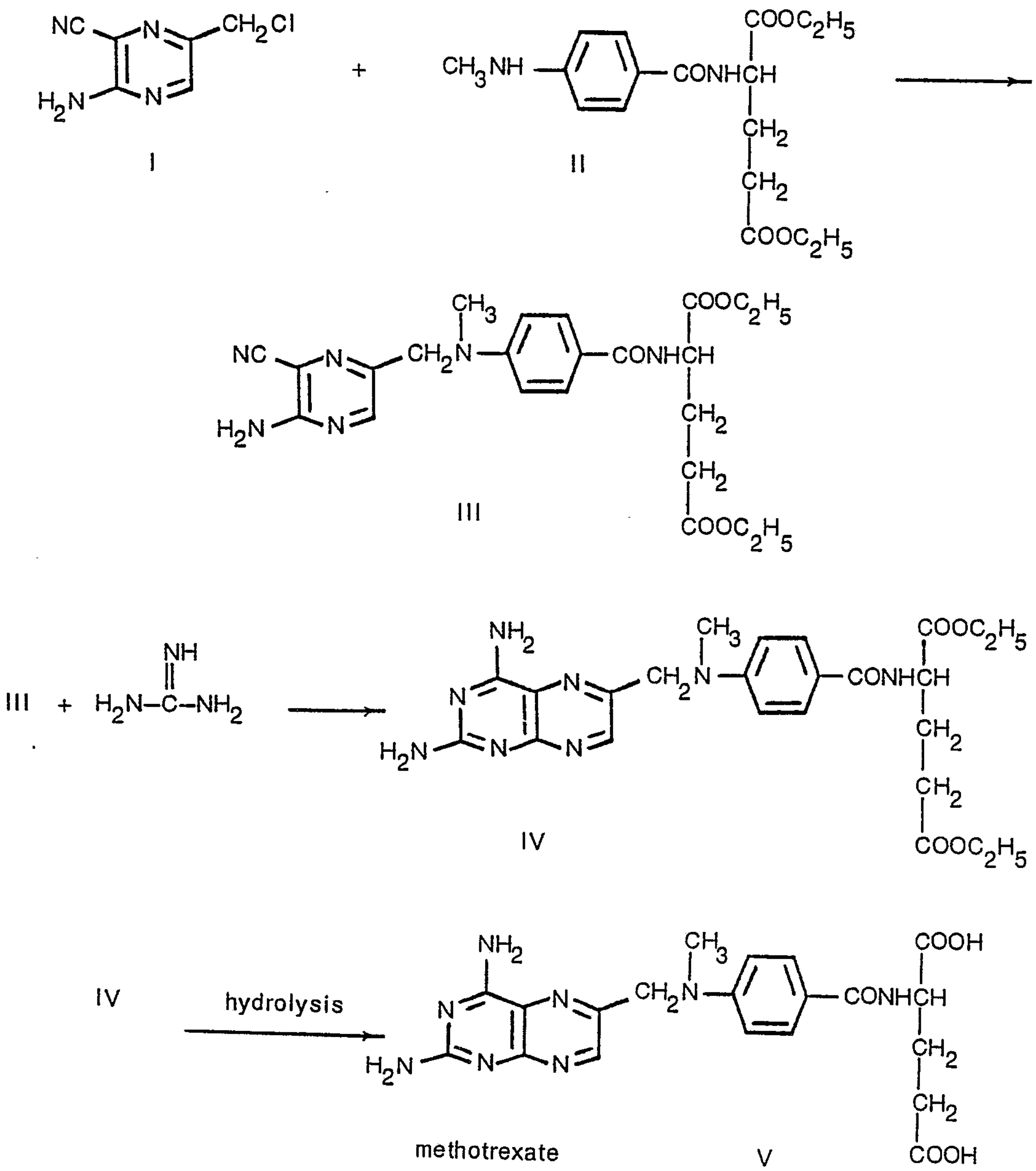


I II III IV methotrexate V

Unfortunately, the reaction of optically active III with guanidine to form IV results in complete racemisation of the side chain (the glutamic acid moiety).

In J. Org. Chem. *40,* 3447—3448 (1975), H.G. Mautner and Young-Ho Kim deal with the synthesis of analogues of folic acid through the cyclisation of substituted 2-amino-3-cyano-pyrazines and state that the problem of side chain racemisation during the guanidine cyclisation cannot be ignored since it affects the biological interactions of the products. The authors mention that the racemisation problem in synthesising folic acid analogues by use of the Taylor synthesis can be avoided by carrying out the cyclisation of the pteroic acid level and then forming the amide carrying the optically active substituent.

However, the attempt to solve the problem in that way would itself constitute a basically different route from the "Taylor method".

The present invention relates to a novel method for preparing methotrexate in which the problem of side chain racemisation is solved, with retention of the important advantages of "the Taylor method".

The method of the present invention for preparing methotrexate comprises reacting N-[4-[[(2-amino-3-cyano-5-pyrazinyl)methyl]methylamino]benzoyl]glutamic acid or a monoester thereof or a mixture of N-[4-[[(2-amino-3-cyano-5-pyrazinyl)methyl]methylamino]benzoyl]glutamic acid and a monoester thereof in the L- or D-form with guanidine or a guanidine salt of a weak inorganic or organic aid. It has surprisingly been found that when N-[4-[[(2-amino-3-cyano-5-pyrazinyl)methyl]-methylamino]benzoyl]glutamic acid or a monoester thereof of a mixture of N-[4-[[(2-amino-3-cyano-5-pyrazinyl)methyl]methylamino]benzoyl]glutamic acid and a monoester thereof in L- or D-form is reacted with guanidine or a guanidine salt of a weak inorganic or organic acid, the resulting methotrexate has the same optical form as the corresponding starting material.

The guanidine salt of a weak inorganic or organic acid may, for example, by guanidine carbonate or guanidine acetate. The reaction of the N-[4-[[(2-amino-3-cyano-5-pyrazinyl)methyl]methylamino]-benzoyl]glutamic acid and/or its monoester with guanidine or a guanidine salt of a weak inorganic or organic acid is preferably performed in dimethyl sulfoxide at a temperature of about 100°C.

The resulting methotrexate may be obtained, e.g., by pouring the reaction mixture into water at pH 4 and separating the methotrexate by filtration. The methotrexate is obtained in high yield. A conventional acid/base purification gives methotrexate of good purity.

It should be noted that in order to avoid change of the optical form during the reaction with guanidine, the N-[4-[[(2-amino-3-cyano-5-pyrazinyl)methyl]methylamino]benzoyl]glutamic acid should be free from any content of the corresponding diester. On the other hand, it has been found that the N-[4-[[(2-amino-3-cyano-5-pyrazinyl)methyl]methylamino]benzoyl]glutamic acid can contain the monoester (half ester) in any amount without effecting the yield or the optical form. Any such content of monoester will be found in the reaction product as the monoester of methotrexate which is then easily hydrolysed during the acid/base purification step. Therefore, instead of or, in combination with, the N-[4-[[(2-amino-3-cyano-5-pyrazinyl)methyl]methylamino]benzoyl]glutamic acid, the corresponding monoester, in particular the corresponding monoethylester may be used.

As indicated above, the N-[4-[[(2-amino-3-cyano-5-pyrazinyl)methyl]methylamino]benzoyl]-glutamic acid may be either the L-form or the D-form, and the methotrexate obtained in the process will correspondingly be L- or D-methotrexate. In view of the established clinical utility of the L-form, this is the preferred form prepared by the method of the invention, but the D-form is contemplated also to be useful in the same field as the L-form.

The N-[4-[[(2-amino-3-cyano-5-pyrazinyl)methyl]methylamino]benzoyl]glutamic acid is believed to be a novel compound and, as such, constitutes an aspect of the present invention. Also the monoesters, for example the monoethyl ester thereof, are believed to be novel and constitute valuable compounds of the present invention.

According to the invention, the N-[4-[[(2-amino-3-cyano-5-pyrazinyl)methyl]methylamino]-benzoyl]glutamic acid may be prepared by alkylation of N-[4-(methylamino)benzoyl]glutamic acid or a salt thereof with 2-amino-3-cyano-5-halomethylpyrazine, in particular 2-amino-3-cyano-5-chloromethylpyrazine. In this way, the N-[4-[[(2-amino-3-cyano-5-pyrazinyl)methyl]methylamino]benzoyl]-glutamic acid is obtained in good yield and in the same optical form as the corresponding N-[4-(methylamino)benzoyl]glutamic acid. This reaction is preferably conducted in a mixture of water and organic solvent such as methanol, tetrahydrofuran, or acetonitrile, at a pH between 5 and 11.

When the starting compound is prepared in this manner, the reaction sequence leading to methotrexate can be illustrated as follows:

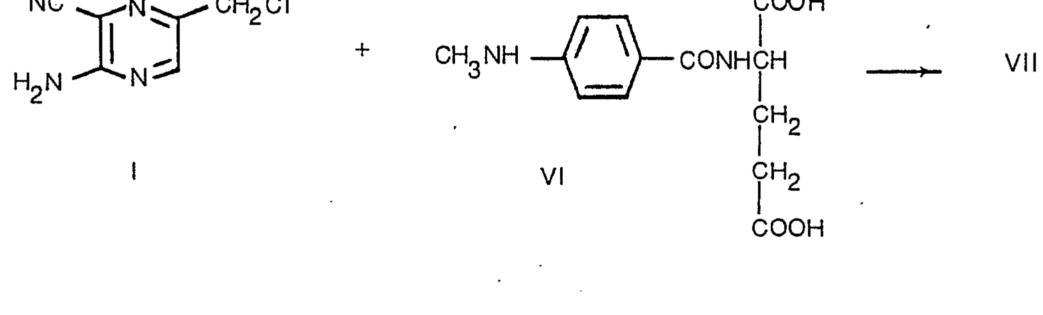

I

VI

VII

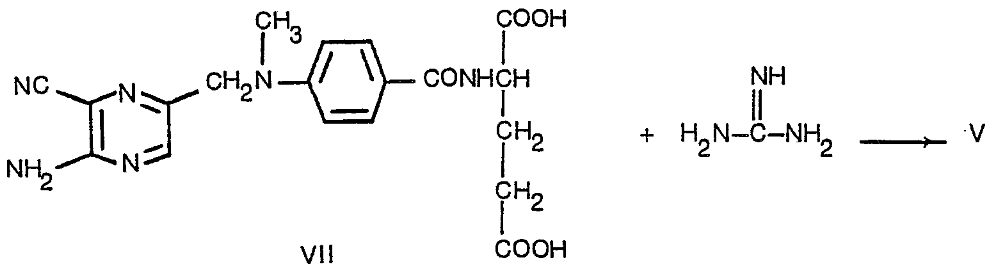

VII

N-[4-(Methylamino)benzoyl]glutamic acid is a known compound, vide, e.g., S.-C. J. Fu and Marion Reiner, J. Org. Chem. *30*, p. 1277 (1965).

Another method for preparing the N-[4-[[(2-amino-3-cyano-5-pyrazinyl)methyl]methylamino]-benzoyl]glutamic acid in the L- or D-form comprises hydrolysing N-[4-[[(2-amino-3-cyano-5-pyrazinyl)-methyl]methylamino]benzoyl]glutamic acid diethylester in the L- or D-form. This hydrolysis should be conducted with care because of the reactive nitrile group in position 3 of the pyrazine. The hydrolysis is preferably performed at or below room temperature in a mixture of water and an organic solvent, for example acetonitrile.

When the starting compound is prepared in this manner, the complete synthesis of methotrexate can be illustrated as follows:

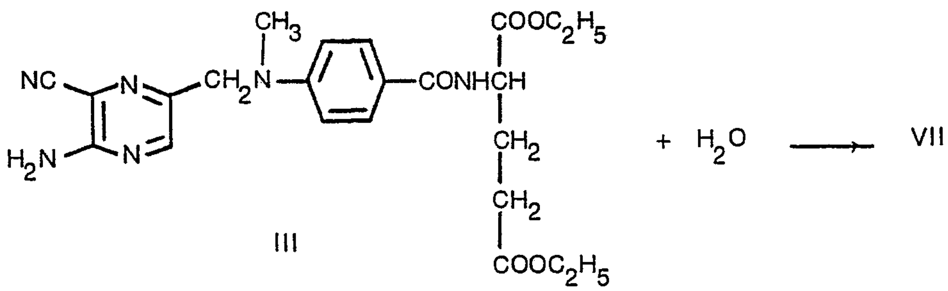

III

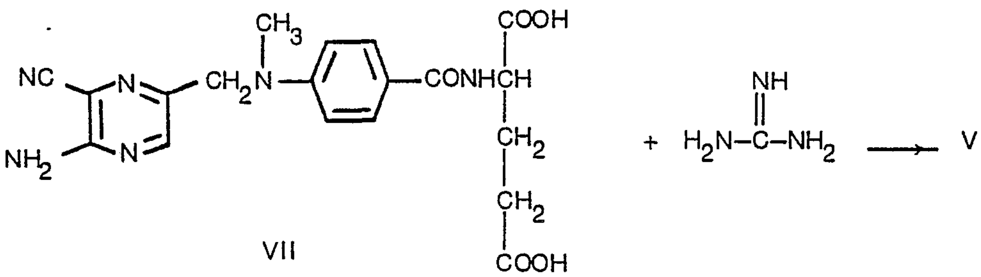

VII

Irrespective of in which way the starting compound is prepared, the method of the present invention results in a good yield of methotrexate which is the same optical form as the corresponding starting material and of good purity.

U.S. Patent No. 4,057,548 claims the preparation of a compound having the formula

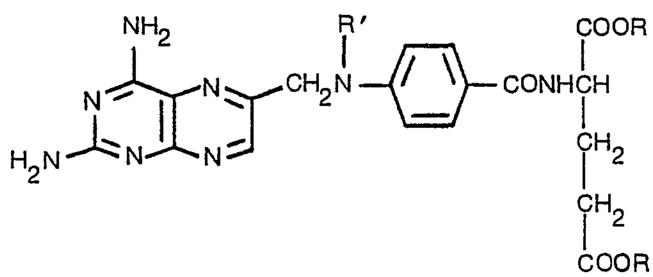

wherein R is hydrogen or a lower alkyl group and R′ is hydrogen or methyl, by treating a compound having a formula

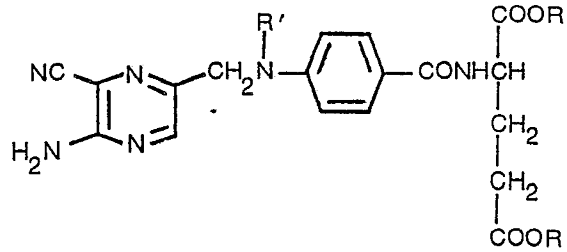

wherein R is hydrogen or a lower alkyl group and R′ is hydrogen or methyl, with a guanidine salt of a weak organic acid. The patent, however, does not disclose N-[4-[[(2-amino-3-cyano-5-pyrazinyl)-methyl]methylamino]benzoyl]glutamic acid and, in particular, does not disclose or indicate the preparation of optically active methotrexate by reacting optically active N-[4-[[(2-amino-3-cyano-5-pyrazinyl)-methyl]methylamino]benzoyl]glutamic acid with guanidine or a guanidine salt of a weak organic acid. The only example of the preparation of methotrexate given in the patent utilizes the diethylester of N-[4-[[(2-amino-3-cyano-5-pyrazinyl)methyl]methylamino]benzoyl]glutamic acid and states that the resulting methotrexate is completely racemic.

The invention is further illustrated by the following examples.

Example 1

N-[4-[[(2-Amino-3-cyano-5-pyrazinyl)methyl]methylamino]benzoyl]-L-glutamic acid (VII)

3.4 g (0.02 mol) 2-amino-3-cyano-5-chloromethylpyrazine (I), 6.5 g (0.02 mol) sodium N-[4-(methylamino)benzoyl]-L-glutamate (VI) and 2.8 g (0.02 mol) potassium carbonate were reached in a mixture of acetonitrile (100 ml) and water (100 ml) for 3 hours at 17°C.

10 g of sodium chloride were added and the reaction mixture was extracted once with ethyl acetate. The lower phase was acidified to about pH 4 with 3N hydrochloric acid and extracted twice with ethyl acetate. The combined organic phases were washed twice with 15% (w/w) sodium chloride in water solution.

The organic phase was dried with anhydrous $MgSO_4$, and evaporated to give a yellow semicrystalline product, which was used directly in the next step. The yield was 6.2 g (75%). A sample was stirred with diethyl ether, filtered, and dried to give a light, yellow product, which melted to a glass at 75—77°C. the glass was converted into a yellow melt at 122—124°C.

$[\alpha]^{D}_{20} = +21° \pm 1°$ (c = 1 in 0.1N NaOH).

The product is about 90% pure, according to HPLC.

Example 2

N-[4-[[(2-Amino-3-cyano-5-pyrazinyl)methyl]methylamino]benzoyl]-L-glutamic acid (VII)

3.4 g (7.2 mmol) N-[4-[[(2-amino-3-cyano-5-pyrazinyl)methyl]methylamino]benzoyl]-L-glutamic acid diethylester (III), 144 ml acetonitrile and 144 ml 0.1N sodium hydroxide (14.4 mmol) were stirred at 5°C for 1 hour. The reaction mixture was acidified to about pH 4 with 3N hydrochloric acid and extracted three times with ethyl acetate. The combined organic phases were washed twice with water, dried with anhydrous $MgSO_4$, and evaporated to give a yellow semicrystalline product, which was used directly in the next step. The yield was 2.4 g (80%). A sample was stirred with diethyl ether, filtered and dried to give a light, yellow product, which melted to a glass at 74—76°C. The glass was converted into a yellow melt at 94—97°C.

$[\alpha]^{D}_{20} = +21° \pm 1°$ (c = 1 in 0.1N NaOH). According to HPLC the product consists of about 80% diacid, about 18% monoester, and 1—2% impurities.

Example 3

N-[4-[[(2,4-Diamino-6-pteridinyl)methyl]methylamino]benzoyl]-L-glutamic acid (L-methotrexate) (V).

2.0 g (5 mmol) VII from Example 2, 30 ml DMSO, 1.2 ml acetic acid (5 mmol) and 3.6 g (40 mmol) guanidine carbonate were mixed and reacted at 95—100°C for 1 hour. The clear, yellow reaction mixture was cooled and poured into water (100 ml). The slightly unclear solution was filtered and acidified with mineral acid to pH 4. The resulting slurry was cooled to 10°C and filtered. The filter cake was washed with water and ethanol.

The wet cake was stirred with water (40 ml) and pH brought to 12.5 with 3N NaOH. The mixture was stirred at this pH at 20°C for $\frac{1}{2}$ hour. Charcoal was added. The mixture was filtered and the filtrate acidified with mineral acid to pH 4. The resulting slurry was cooled to 10°C and filtered. The

filter cake was washed with water and finally with ethanol. The wet yellow crystals were dried at 25°C. The yield was 1.8 g (72%).

Calc. for $C_{20}H_{22}N_8O_5$ + 8.0% $H_2O$ + 2.0% HCl:

| | | | | | |
|---|---|---|---|---|---|
| | C 47.6% | H 5.3% | N 22.2% | Cl 2.0% | $H_2O$ 8.0% |
| Found: | C 47.2% | H 5.5% | N 22.5% | Cl 2.0% | $H_2O$ 8.0% (KF) |

HPLC indicates a good purity of methotrexate when compared with an authentic sample.

$[\alpha]^D_{20} = +20° \pm 1°$ (c = 1 in 0.1N NaOH). The IR (KBr) spectrum is consistent with the structure of methotrexate.

Example 4

N-[4-[[(2,4-Diamino-6-pteridinyl)methyl]methylamino]benzoyl]-D-glutamic acid (D-methotrexate) (V).

The reaction in Example 2 was repeated with N-[4-[[(2-amino-3-cyano-5-pyrazinyl)-methyl]methylamino]benzoyl]-D-glutamic acid diethylester substituted for the L-isomer. Then the raw product was reacted as in Example 3 to give D-methotrexate $[\alpha]^D_{20} = -20° \pm 1°$ (c = 1 in 0.1N NaOH). The compound was analytically pure within ± 0.4% abs. HPLC indicates a good purity of methotrexate when compared with an authentic sample.

The IR (KBr) spectrum is consistent with the structure of methotrexate.

**Claims**

1. A method for preparing L- or D-methotrexate, comprising reacting N-[4-[[(2-amino-3-cyano-5-pyrazinyl)methyl]methylamino]benzoyl]glutamic acid or a monoester thereof or a mixture of N-[4-[[(2-amino-3-cyano-5-pyrazinyl)methyl]methylamino]benzoyl]glutamic acid and a monoester thereof, said starting compound(s) being in the L- or D-form, with guanidine or a guanidine salt of a weak inorganic or organic acid.

2. A method as claimed in claim 1 in which said starting compound(s) is (are) substantially free from any content of the corresponding diester.

3. A method as claimed in claims 1 and 2 in which the reaction is conducted in dimethyl sulfoxide at a temperature of about 100°C.

4. A method as claimed in claim 1 or 2 in which the working up of the methotrexate is performed by acid/base purification.

5. A method as claimed in any of the preceding claims in which the N-[4-[[(2-amino-3-cyano-5-pyrazinyl)methyl]methylamino]benzoyl]glutamic acid is prepared by alkylation of N-[4-(methylamino)-benzoyl]glutamic acid or a salt thereof with 2-amino-3-cyano-5-halomethylpyrazine, the N-[4-(methylamino)benzoyl]glutamic acid being in the same optical form as the desired reaction product.

6. A method as claimed in claim 5 in which the alkylation is performed in a mixture of water and an organic solvent at a pH between 5 and 11.

7. A method as claimed in any of claims 1—4 in which the N-[4-[[(2-amino-3-cyano-5-pyrazinyl)-methyl]methylamino]benzoyl]glutamic acid is prepared by hydrolysis of N-[4-[[(2-amino-3-cyano-5-pyrazinyl)methyl]methylamino]benzoyl]glutamic acid diethylester, the N-[4-[[(2-amino-3-cyano-5-pyrazinyl)methyl]methylamino]benzoyl]glutamic acid diethylester being in the same optical form as the desired reaction product.

8. A method as claimed in claim 7 in which the hydrolysis is performed at or below room temperature in a mixture of water and an organic solvent.

9. A method as claimed in any of the preceding claims in which the L-isomer of methotrexate is prepared.

10. A method as claimed in any of claims 1—8 in which the D-isomer of methotrexate is prepared.

11. N-[4-[[(2-Amino-3-cyano-5-pyrazinyl)methyl]methylamino]benzoyl]glutamic acid and monoesters thereof in the L- or D-form.

12. N-[4-[[(2-Amino-3-cyano-5-pyrazinyl)methyl]methylamino]benzoyl]glutamic acid monoethylester in the L- or D-form.

13. A method for preparing N-[4-[[(2-amino-3-cyano-5-pyrazinyl)methyl]methylamino]benzoyl]-glutamic acid in the L- or D-form, comprising alkylating N-[4-(methylamino)benzoyl]glutamic acid in the L- or D-form or a salt thereof with 2-amino-3-cyano-5-halomethylpyrazine.

14. A method for preparing N-[4-[[(2-amino-3-cyano-5-pyrazinyl)methyl]methylamino]benzoyl]-glutamic acid in the L- or D-form, comprising hydrolysing N-[4-[[(2-amino-3-cyano-5-pyrazinyl)-methyl]methylamino]benzoyl]glutamic acid diethylester in the L- or D-form.

**Revendications**

1. Procédé de préparation du méthotrexate L ou D, comprenant la réaction de l'acide N-[4-[[(2-amino-3-cyano-5-pyrazinyl)méthyl]méthylamino]benzoyl]-glutamique ou d'un de ses monoesters ou d'un mélange d'acide N-[4-[[(2-amino-3-cyano-5-pyrazinyl)méthyl]méthylamino]benzoyl]-glutamique et d'un de ses monoesters, le(s)dit(s) composé(s) de départ étant sous forme L ou D, avec la guanidine ou un sel de guanidine et d'un acide faible minéral ou organique.

2. Procédé selon la revendication 1, caractérisé en ce que le(s)dit(s) composé(s) de départ est (sont) sensiblement exempt(s) de toute teneur en diester correspondant.

3. Procédé selon les revendications 1 et 2, caractérisé en ce que la réaction est réalisée dans le diméthylsulfoxyde à une température d'environ 100°C.

4. Procédé selon la revendication 1 ou 2, caractérise en ce que l'élaboration du méthotrexate s'effectue par purification acide/base.

5. Procédé selon l'une quelconque des revendications précédentes, caractérisé en ce que l'acide selon la revendication 1 ou 2, caractérisé en ce que l'élaboration du méthotrexate d'effectue par purification acide/base.

5. Procédé selon l'une quenconque des revendications précédentes, caractérisé en ce que l'acide N-[4-[[(2-amino-3-cyano-5-pyrazinyl)méthyl]méthylamino]benzoyl]-glutamique est préparé par alkylation de l'acide N-[4-(méthylamino)benzoyl]-glutamique ou d'un de ses sels avec la 2-amino-3-cyano-5-halogénométhylpyrazine, l'acide N-[4-(méthylamino)benzoyl]-glutamique ayant la même forme optique que le produit de réaction désiré.

6. Procédé selon la revendication 5, caractérisé en ce que l'alkylation est réalisée dans un mélange d'eau et de solvant organique à un pH compris entre 5 et 11.

7. Procédé selon l'une quelconque des revendications 1 à 4, caractérisé en ce que l'acide N-[4-[[(2-amino-3-cyano-5-pyrazinyl)méthyl]méthylamino]benzoyl]-glutamique est préparé par hydrolyse de l'ester diéthylique de l'acide N-[4-[[(2-amino-3-cyano-5-pyrazinyl)méthyl]méthylamino]benzoyl]-glutamique, l'ester diéthylique de l'acide N-[4-[[(2-amino-3-cyano-5-pyrazinyl)méthyl]méthylamino]-benzoyl]-glutamique ayant la même forme optique que le produit de réaction désiré.

8. Procédé selon la revendication 7, caractérise en ce que l'hydrolyse est réalisée à la température ambiante ou à une température inférieure dans un mélange d'eau et de solvant organique.

9. Procédé selon l'une quelconque des revendications précédentes, caractérisé en ce que l'on prépare l'isomère L du méthotrexate.

10. Procédé selon l'une quelconque des revendications 1 à 8, caractérisé en ce que l'on prépare l'isomère D du méthotrexate.

11. Acide N-[4-[[(2-amino-3-cyano-5-pyrazinyl)méthyl]méthylamino]benzoyl]-glutamique et ses monoesters de forme L ou D.

12. Ester monoéthylique de l'acide N-[4-[[(2-amino-3-cyano-5-pyrazinyl)méthyl]méthylamino]-benzoyl]-glutamique de forme L ou D.

13. Procédé de préparation de l'acide N-[4-[[(2-amino-3-cyano-5-pyrazinyl)méthyl]méthylamino]benzoyl]-glutamique sous la forme L ou D, comprenant l'alkylation de l'acide N-[4-(méthylamino)benzoyl]-glutamique de forme L ou D ou d'un de ses sels avec la 2-amino-3-cyano-5-halogénométhylpyrazine.

14. Procédé de préparation de l'acide N-[4-[[(2-amino-3-cyano-5-pyrazinyl)méthyl]méthylamino]benzoyl]-glutamique sous la forme L ou D, comprenant l'hydrolyse de l'ester diéthylique de l'acide N-[4-[[(2-amino-3-cyano-5-pyrazinyl)méthyl]méthylamino]benzoyl]-glutamique de forme L ou D.

**Patentansprüche**

1. Verfahren zur Herstellung von L- oder D-Methotrexat, umfassend das Umsetzen von N-[4-[[(2-Amino-3-cyano-5-pyrazinyl)methyl]methylamino]benzoyl]glutaminsäure oder eines Monoesters hiervon oder einer Mischung von N-[4-[[(2-Amino-3-cyano-5-pyrazinyl)methyl]methylamino]benzoyl]-glutaminsäure und eines Monoesters hiervon, wobei die Ausgangsverbindung bzw. -verbindungen in der L- oder D-Form vorliegt bzw. vorliegen, mit Guanidin oder einem Guanidinsalz einer schwachen, anorganischen oder organischen Säure.

2. Verfahren nach Anspruch 1, dadurch gekennzeichnet, daß die Ausgangsverbindung(en) von irgendeinem Gehalt des korrespondierenden Diesters im wesentlichen frei ist (sind).

3. Verfahren nach Anspruch 1 und 2, dadurch gekennzeichnet, daß die Umsetzung in Dimethylsulfoxid bei einer Temperatur von etwa 100°C durchgeführt wird.

4. Verfahren nach Anspruch 1 oder 2, dadurch gekennzeichnet, daß die Aufarbeitung des Methotrexats durch Säure/Base-Reinigung durchgeführt wird.

5. Verfahren nach mindestens einem der vorangehenden Ansprüche, dadurch gekennzeichnet, daß die N-[4-[[(2-Amino-3-cyano-5-pyrazinyl)methyl]methylamino]benzoyl]glutaminsäure durch Alkylierung von N-[4-(Methylamino)benzoyl]glutaminsäure oder eines Salzes hiervon mit 2-Amino-3-cyano-5-halogenmethylpyrazin hergestellt wird, wobei die N-[4-(Methylamino)benzoyl]glutaminsäure in der gleichen optischen Form wie das erwünschte Reaktionsprodukt vorliegt.

6. Verfahren nach Anspruch 5, dadurch gekennzeichnet, daß die Alkylierung in einer Mischung aus Wasser und einem organischen Lösungsmittel bei einem pH zwischen 5 und 11 durchgeführt wird.

7. Verfahren nach mindestens einem der Ansprüch 1 bis 4, dadurch gekennzeichnet, daß die N-[4-[[(2-Amino-3-cyano-5-pyrazinyl)methyl]methylamino]benzoyl]glutaminsäure durch Hydrolyse von N-[4-[[(2-Amino-3-cyano-5-pyrazinyl)methyl]methylamino]benzoyl]glutaminsäurediethylester hergestellt wird, wobei der N-[4-[[(2-Amino-3-cyano-5-pyrazinyl)methyl]methylamino]benzoyl]glutaminsäurediethylester in der gleichen optischen Form wie das erwünschte Reaktionsprodukt vorliegt.

8. Verfahren nach Anspruch 7, dadurch gekennzeichnet, daß die Hydrolyse bei oder unterhalb Raumtemperatur in einer Mischung aus Wasser und einem organischen Lösungsmittel durchgeführt wird.

9. Verfahren nach mindestens einem der vorangehenden Ansprüche, dadurch gekennzeichnet, daß das L-Isomer von Methotrexat hergestellt wird.

10. Verfahren nach mindestens einem der Ansprüche 1 bis 8, dadurch gekennzeichnet, daß das D-Isomer von Methotrexat hergestellt wird.

11. N-[4-[[(2-Amino-3-cyano-5-pyrazinyl)methyl]methylamino]benzoyl]glutaminsäure und Monoester hiervon in der L- oder D-Form.

12. N-[4-[[(2-Amino-3-cyano-5-pyrazinyl)methyl]methylamino]benzoyl]glutaminsäuremonoethylester in der L- oder D-Form.

13. Verfahren zur Herstellung von N-[4-[[(2-Amino-3-cyano-5-pyrazinyl)methyl]methylamino]-benzoyl]glutaminsäure in der L- oder D-Form, umfassend das Alkylieren von N-[4-(Methylamino)-benzoyl]glutaminsäure in der L- oder D-Form oder eines Salzes hiervon mit 2-Amino-3-cyano-5-halogenmethylpyrazin.

14. Verfahren zur Herstellung von N-[4-[[(2-Amino-3-cyano-5-pyrazinyl)methyl]methylamino]-benzoyl]glutaminsäure in der L- oder D-Form, umfassend das Hydrolysieren von N-[4-[[(2-Amino-3-cyano-5-pyrazinyl)methyl]methylamino]benzoyl]glutaminsäurediethylester in der L- oder D-Form.